# EUROPEAN PATENT APPLICATION

(11) **EP 2 942 396 A1**
(43) Date of publication of application: **11.11.2015**
(21) Application number: 14167449.9
(22) Date of filing: 07.05.2014
(51) Int. Cl.: C12P 19/04, A61K 39/09, C07K 14/315, C08B 37/00

(54) **Polysaccharides produced by CPSC mutants**

(71) Applicant: Novartis AG, 4056 Basel (CH)
(72) Inventor: The designation of the inventor has not yet been filed
(74) Representative: Evans, Stephen John Eves

(57) **Abstract**

The invention relates to bacterial mutants, particularly from *Streptococcus agalactiae,* of CpsC that produce low molecular weight capsular polysaccharides. The polysaccharides are useful for producing vaccines comprising the polysaccharides alone or conjugated to proteins.

SEQUENCE LISTING

<110>  Novartis AG

<120>  POLYSACCHARIDES PRODUCED BY CPSC MUTANTS

<130>  PAT056260-EP-EPA

<160>  10

<170>  PatentIn version 3.5

<210>  1
<211>  230
<212>  PRT
<213>  Streptococcus agalactiae

<400>  1

Met Asn Lys Ile Ala Asn Thr Glu Val Glu Ile Asn Ile Phe Asn Leu
1               5                   10                  15

Leu Lys Lys Leu Trp Lys Lys Lys Phe Leu Ile Ile Phe Val Ala Ile
            20                  25                  30

Ala Phe Ala Thr Ala Gly Leu Phe Tyr Arg Leu Phe Ile Val Thr Pro
        35                  40                  45

Gln Tyr Thr Ser Ser Thr Arg Ile Tyr Val Ser Lys Pro Thr Leu Pro
    50                  55                  60

Asn Asn Ser Ile Thr Val Gln Asp Leu Gln Ala Gly Ser Phe Leu Ala
65                  70                  75                  80

Asn Asp Tyr Lys Glu Ile Ile Thr Ser Thr Asp Val Leu Glu Lys Val
                85                  90                  95

Ile Ser Ser Glu Lys Leu Asn Tyr Pro Ser Ser Gln Leu Leu Gln Lys
            100                 105                 110

Ile Thr Val Ser Ile Leu Lys Gly Thr Arg Val Ile Ser Ile Ser Val
        115                 120                 125

Glu Asp Val Asn Pro Lys Met Ser Gln Lys Ile Ala Asn Ser Val Arg
    130                 135                 140

Glu Ala Ala Val Ser Lys Ile Lys Ala Val Thr Gln Val Glu Asp Ile
145                 150                 155                 160

Thr Thr Ile Glu Lys Gly Asn Leu Pro Lys Ser Pro Ser Ser Pro Asn
                165                 170                 175

Ile Lys Lys Asn Val Leu Ile Trp Phe Ile Val Gly Ala Gly Leu Ser
            180                 185                 190

Thr Ile Val Leu Val Ile Met Gly Ile Leu Asp Asp Arg Val Asn Thr
        195                 200                 205

Glu Glu Asp Ile Glu Lys Val Leu Gly Leu Thr Ser Leu Gly Ile Val
    210                 215                 220

Pro Asp Leu Asn Lys Leu
225                 230

<210>  2
<211>  693
<212>  DNA
<213>  Streptococcus agalactiae

<400>  2
atgaataaaa tagctaatac agaagttgaa ataaatatat tcaacctttt aaaaaaattg      60

tggaaaaaga aatttttaat catatttgtt gccattgcgt ttgcaacagc tggacttttc      120

tatagacttt ttattgttac gccacaatat acttcttcaa caaggatata tgtcagtaaa      180

cctactctcc ctaataatag tattactgta caagatctac aagcagggag ttttcttgca      240

aatgactata aggagattat tacgtctact gacgttctag aaaaagttat ttcttctgaa      300

aaattgaatt atccttcgtc tcagttgctg caaaaaataa cagtttctat tttaaaaggt      360

acacgtgtta tttcaatatc ggtcgaagat gttaatccaa aaatgtctca aaaaatagca      420

aattcagtta gagaagcagc agtttcaaaa atcaaggcag ttactcaagt agaggatatc      480

actactattg agaagggaaa tttacctaaa tcaccatctt ctcctaatat taaaaagaat      540

gtactaatct ggtttattgt tggtgcagga ttatcaacta ttgttttagt tattatgggt      600

attttggatg atcgtgttaa tactgaagaa gatattgaaa aggtcctagg acttacttct      660

ttaggtatag taccagattt gaataaactt taa                                  693

<210>  3
<211>  135
<212>  PRT
<213>  Artificial Sequence

<220>
<223>  Ext Domain of CpsC

<400>  3

Val Thr Pro Gln Tyr Thr Ser Ser Thr Arg Ile Tyr Val Ser Lys Pro
1               5                   10                  15

Thr Leu Pro Asn Asn Ser Ile Thr Val Gln Asp Leu Gln Ala Gly Ser
            20                  25                  30

Phe Leu Ala Asn Asp Tyr Lys Glu Ile Ile Thr Ser Thr Asp Val Leu
        35                  40                  45

Glu Lys Val Ile Ser Ser Glu Lys Leu Asn Tyr Pro Ser Ser Gln Leu
    50                  55                  60

Leu Gln Lys Ile Thr Val Ser Ile Leu Lys Gly Thr Arg Val Ile Ser
65                  70                  75                  80

Ile Ser Val Glu Asp Val Asn Pro Lys Met Ser Gln Lys Ile Ala Asn
                85                  90                  95

Ser Val Arg Glu Ala Ala Val Ser Lys Ile Lys Ala Val Thr Gln Val
            100                 105                 110

Glu Asp Ile Thr Thr Ile Glu Lys Gly Asn Leu Pro Lys Ser Pro Ser
        115                 120                 125

Ser Pro Asn Ile Lys Lys Asn
    130                 135

<210>  4
<211>  45
<212>  PRT
<213>  Artificial Sequence

<220>
<223>  H2N Intracellular Domain plus TM Domain

<400>  4

Met Asn Lys Ile Ala Asn Thr Glu Val Glu Ile Asn Ile Phe Asn Leu
1               5                   10                  15

Leu Lys Lys Leu Trp Lys Lys Lys Phe Leu Ile Ile Phe Val Ala Ile
            20                  25                  30

Ala Phe Ala Thr Ala Gly Leu Phe Tyr Arg Leu Phe Ile
        35                  40                  45

<210>  5
<211>  50
<212>  PRT
<213>  Artificial Sequence

<220>
<223>  COOH Intracellular Domain plus TM Domain

<400>  5

Val Leu Ile Trp Phe Ile Val Gly Ala Gly Leu Ser Thr Ile Val Leu

1               5                   10                  15

Val Ile Met Gly Ile Leu Asp Asp Arg Val Asn Thr Glu Glu Asp Ile
            20                  25                  30

Glu Lys Val Leu Gly Leu Thr Ser Leu Gly Ile Val Pro Asp Leu Asn
        35                  40                  45

Lys Leu
    50

<210>  6
<211>  24
<212>  PRT
<213>  Artificial Sequence

<220>
<223>  H2N Intracellular Domain minus TM domain

<400>  6

Met Asn Lys Ile Ala Asn Thr Glu Val Glu Ile Asn Ile Phe Asn Leu
1               5                   10                  15

Leu Lys Lys Leu Trp Lys Lys Lys
            20

<210>  7
<211>  28
<212>  PRT
<213>  Artificial Sequence

<220>
<223>  COOH Intracellular Domain minus TM domain

<400>  7

Asp Asp Arg Val Asn Thr Glu Glu Asp Ile Glu Lys Val Leu Gly Leu
1               5                   10                  15

Thr Ser Leu Gly Ile Val Pro Asp Leu Asn Lys Leu
            20                  25

<210>  8
<211>  99
<212>  PRT
<213>  Artificial Sequence

<220>
<223>  WZZ Chain Length Determinant Protein Motif

<400>  8

Thr Pro Gln Tyr Thr Ser Ser Thr Arg Ile Tyr Val Ser Lys Pro Thr
1               5                   10                  15

Leu Pro Asn Asn Ser Ile Thr Val Gln Asp Leu Gln Ala Gly Ser Phe
            20                  25                  30

Leu Ala Asn Asp Tyr Lys Glu Ile Ile Thr Ser Thr Asp Val Leu Glu
        35                  40                  45

Lys Val Ile Ser Ser Glu Lys Leu Asn Tyr Pro Ser Ser Gln Leu Leu
    50                  55                  60

Gln Lys Ile Thr Val Ser Ile Leu Lys Gly Thr Arg Val Ile Ser Ile
65                  70                  75                  80

Ser Val Glu Asp Val Asn Pro Lys Met Ser Gln Lys Ile Ala Asn Ser
                85                  90                  95

Val Arg Glu

<210> 9
<211> 61
<212> PRT
<213> Artificial Sequence

<220>
<223> Nuf2 family sequence motif

<400> 9

Ser Phe Leu Ala Asn Asp Tyr Lys Glu Ile Ile Thr Ser Thr Asp Val
1               5                   10                  15

Leu Glu Lys Val Ile Ser Ser Glu Lys Leu Asn Tyr Pro Ser Ser Gln
            20                  25                  30

Leu Leu Gln Lys Ile Thr Val Ser Ile Leu Lys Gly Thr Arg Val Ile
        35                  40                  45

Ser Ile Ser Val Glu Asp Val Asn Pro Lys Met Ser Gln
    50                  55                  60

<210> 10
<211> 68
<212> PRT
<213> Artificial Sequence

<220>
<223> Domain of Unknown Function DUF59 motif

<400> 10

Asp Val Leu Glu Lys Val Ile Ser Ser Glu Lys Leu Asn Tyr Pro Ser

1               5                   10                  15

Ser Gln Leu Leu Gln Lys Ile Thr Val Ser Ile Leu Lys Gly Thr Arg
            20                  25                  30

Val Ile Ser Ile Ser Val Glu Asp Val Asn Pro Lys Met Ser Gln Lys
        35                  40                  45

Ile Ala Asn Ser Val Arg Glu Ala Ala Val Ser Lys Ile Lys Ala Val
    50                  55                  60

Thr Gln Val Glu
65

## Description

### FIELD OF THE INVENTION

The present invention relates to methods for extracting and isolating capsular polysaccharides (CPS) from bacteria that comprise mutations in the capsular polysaccharide synthesis gene CpsC. The extracted polysaccharides are useful for producing vaccines comprising the polysaccharides alone or conjugated to proteins.

### BACKGROUND OF THE INVENTION

In the last 25 years, conjugate vaccines, comprising bacterial capsular polysaccharides (cps) conjugated to protein carriers have been developed. Capsular polysaccharides are important immunogens found on the surface of bacteria involved in various bacterial diseases. This feature has led to them being an important component in the design of vaccines. As saccharides are T-independent antigens, generally cps are poorly immunogenic. Conjugation to a carrier can convert T-independent antigens into T-dependent antigens, thereby enhancing memory responses and allowing protective immunity to develop.

Therefore, the most effective saccharide vaccines are based on glycoconjugates. Examples include, amongst others, the Haemophilus influenzae type b (Hib) conjugate vaccine, conjugate vaccines against Streptococcus pneumoniae and serogroup C Neisseria meningitidis (MenC). Another bacterium for which conjugate vaccines have been described is Streptococcus agalactiae, also known as 'Group B streptococcus', or simply 'GBS'. The 'B' in 'GBS' refers to the Lancefield classification which is based on the antigenicity of a carbohydrate which is soluble in dilute acid and called the C carbohydrate. Lancefield identified 13 types of C carbohydrate (designated A to O) that could be serologically differentiated. The organisms that most commonly infect humans are found in groups A, B, D, and G. Within group B, strains of Streptococcus agalactiae are divided into 10 serotypes (la, Ib, II, III, IV, V, VI, VII, VIII and IX) based on the structure of their polysaccharide capsule.

Group B Streptococcus agalactiae causes serious disease, bacteremia and meningitis, in immunocompromised individuals and in neonates. There are two main types of neonatal GBS infection. Early onset disease occurs within 5 days of birth and is manifested by bacteremia (sepsis or infection of the blood) and pneumonia (an infection of the lungs). Late onset disease occurs from the first week of birth up to around three months after birth. Late onset disease is commonly characterized by meningitis (infection of the fluid and lining around the brain) although bacteremia and pneumonia may also occur. GBS colonises the vagina of about 25 percent of young women and is contracted vertically as a baby passes through the birth canal. Approximately 1 percent of infants born via a vaginal birth to colonised mothers will become infected with a mortality rate of between 50-70 percent.

Investigations have been conducted into the development of protein-based and polysaccharide-based vaccines against GBS. Conjugates of each of the capsular polysaccharides vaccines from GBS serotypes la, Ib, II, III, and V have been shown to be safe and immunogenic in humans. For example, vaccination of pregnant women with type III cps has been demonstrated to reduce the incidence of the late onset meningitis - infants acquire protective antibodies via placental transfer and are passively immunised.

For some applications, smaller polysaccharides can improve ease of handling, lower production costs and enhance immunogenicity. In some circumstances, high molecular weight polysaccharides may form insoluble gels when crosslinked. Reducing the chain length of the starting polysaccharide can avoid this problem.

Prior methods of depolymerizing polysaccharides have generally relied on enzymatic methods or on acid hydrolysis which may alter the antigenicity of the polysaccharide due to removal of the labile terminal sialic acid groups. For example, as disclosed in WO98/42718, WO99/18121 and WO2013/050300.

Two further methods (Ozonolysis: Wang Y, Hollingsworth RI, Kasper DL. Proc Natl Acad Sci USA. 1998 Jun 9;95(12):6584-9. Chemical Treatment: Michon F, Uitz C, Sarkar A, D'Ambra AJ, Laude-Sharp M, Moore S, Fusco PC. Clin Vaccine Immunol. 2006 Aug;13(8):936-43) have been used to produce shorter oligosaccharides, of GBS capsular polysaccharides from serotypes II and III. However, these methods are not necessarily applicable to capsular polysaccharides from other GBS serotypes that do not comprise GIcNAc in the backbone.

Accordingly, there is a need for improved procedures which are effective for producing shorter polysaccharides for use in capsular polysaccharide-protein conjugate vaccines.

### SUMMARY OF THE INVENTION

The inventors have discovered a method for producing shorter oligosaccharides of bacterial capsular polysaccharides that does not rely on the use of chemical or other treatment steps to depolymerise the polysaccharide. Advantageously, the method is applicable to capsular polysaccharides of *Streptococcus agalactiae* that do not comprise GIcNAc residues.

Thus, in a first aspect of the invention there is provided a method for producing a low molecular weight capsular polysaccharide the method comprising: culturing a CpsC mutant bacterium in a suitable culture medium and isolating the low molecular weight capsular polysaccharide from the bacterium. The term "low molecular weight", as used herein, means that the capsular polysaccharide produced by the mutant bacterium has an average molecular weight which is less than that produced by an equivalent wild-type strain of the same serotype. Preferably the CpsC mutant bacterium is derived from *Streptococcus agalactiae*, also known as Group B Streptococcus or GBS. Generally the *Streptococcus agalactiae* serotype is selected from the group consisting of la, Ib, II, III, IV, V, VI, VII or VIII, particularly from serotypes la, Ib, II, III or V. The capsular polysaccharide produced by wild type *Streptococcus agalactiae* serotype la has a MW in the range of 150-360 kDa, Wild-type serotype Ib polysaccharides have a MW in the range of 150-300 kDa. Wild-type serotype III polysaccharides have a MW in the range of 50-200 kDa. Wild-type serotype V polysaccharides have a MW of about 100 kDa. Molecular masses can be easily determined by methods known in the art, for example measuring by gel filtration relative to dextran standards. Thus, CpsC mutant bacteria of *Streptococcus agalactiae* may produce capsular polysaccharide having molecular weights less than the molecular weight of the above wild-type capsular polysaccharide. For example, less than 300kDa, less than 250kDa, less than 200kDa, less than 150kDa, less than 100kDa, less than 75kDa, less than 70kDa, less than 65kDa, less than 60kDa, less than 55kDa, less than 50kDa. Particularly between 10kDa and 70kDa, 10kDa and 60kDa, 10kDa and 55kDa or between 10kDa and 50kDa. Capsular polysaccharide produced by CpsC mutant bacterium according to the invention is shorter than the natural full length capsular polysaccharide found in nature and produced by wild-type bacteria.

In certain embodiments, the CpsC mutant bacterium comprises an altered CpsC protein wherein the nucleotide sequence that encodes the CpsC protein (SEQ ID NO:X) is deleted or partially deleted and wherein the CpsC mutant bacterium produces low molecular weight capsular polysaccharide. Particularly the CpsC mutant bacterium comprises an altered CpsC protein wherein the nucleotide sequence that encodes the extracellular domain of the CpsC protein (SEQ ID NO:X) is deleted or partially deleted and wherein the CpsC mutant bacterium produces low molecular weight capsular polysaccharide.

Capsular polysaccharide may be extracted or isolated from CpsC mutant bacteria by methods known in the art. In some embodiments the step of isolating the capsular polysaccharide from the bacterium comprises (i) providing for extraction isolated bacterial cells, concentrated bacterial supernatants from homogenized bacterial cells or pelleted cells; (ii) extracting the capsular polysaccharide from cellular components, particularly by contacting the isolated bacterial cells, concentrated bacterial supernatants from homogenised bacterial cells or pelleted cells with a base reagent; and (iii) separating the extracted capsular polysaccharide from the cellular components to recover the purified capsular polysaccharides.

In particular embodiments step (iii) may comprise removing contaminating nucleic acids and/or proteins from the capsular polysaccharide in aqueous form by the use of alcoholic precipitation, wherein an alcohol and an aqueous metal cation are used to precipitate the nucleic acids and/or proteins leaving the polysaccharide in solution and, separating the precipitated material from the polysaccharide. The method may further comprises one or more steps of diafiltration, for example, after the precipitation of nucleic acids and/or proteins.

In a second aspect, there is provided a low molecular weight capsular polysaccharide from Streptococcus agalactiae, obtainable by the method of the first aspect.

In a third aspect, there is provided an immunogenic composition, particularly a vaccine composition, comprising the low molecular weight capsular polysaccharide of the second aspect.

### BRIEF DESCRIPTION OF THE FIGURES

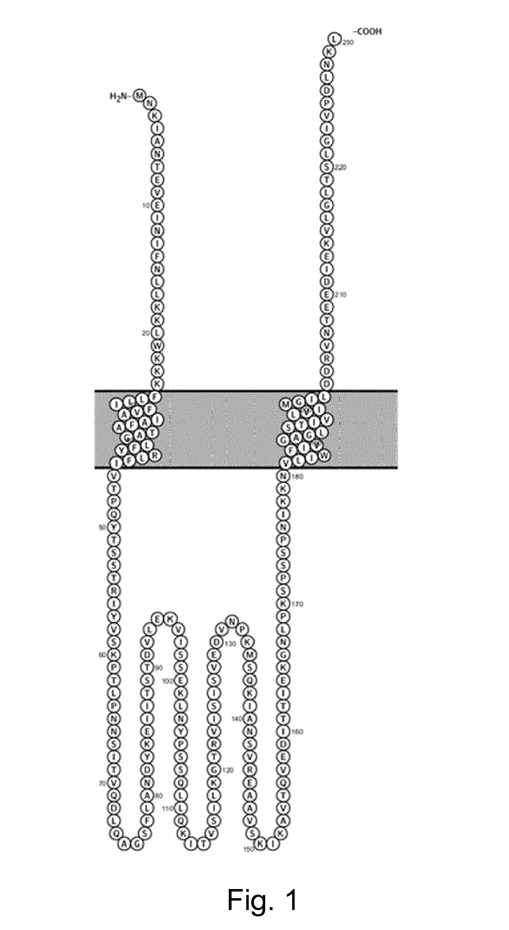

Figure 1: Schematic of the CpsC protein (Accession No. R4Z8W1_STRAG) prepared using Protter (Omasits et al., Bioinformatics. 2013 Nov 21). Intracellular domain - top, extracellular domain - bottom.

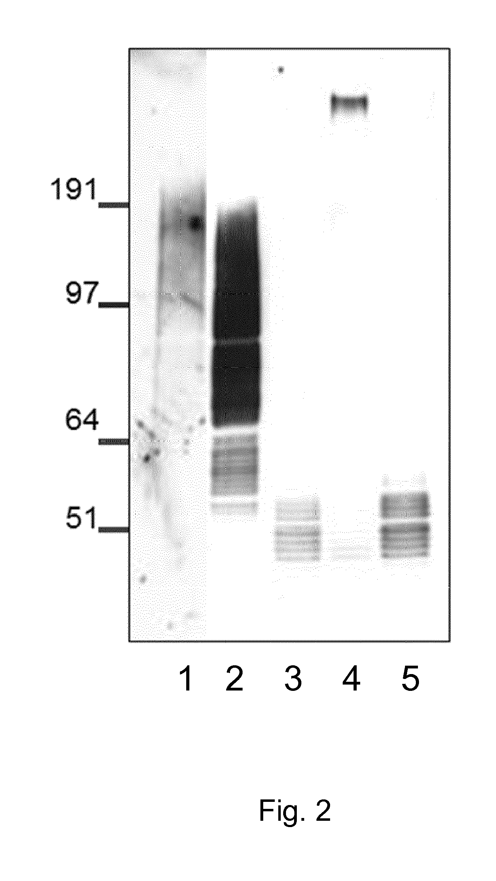

Figure 2: Molecular weight of capsular polysaccharide extracted from (1) Wild-type 515 strain, (2) wild-type 515 strain, (3) ACpsC in 515 background, (4) CpsC AC-term in 515 background and (5) CpsC Δext in 515 background. The MW of mutants in lanes 3 and 5 is significantly lower than those of the wild-type strains.

### DETAILED DESCRIPTION OF THE INVENTION

By introducing mutations into the sequence of the CpsC polynucleotide and/or protein, the present inventors have surprisingly discovered that such bacteria produce capsular polysaccharide that has a shorter length than that produced by wild-type bacteria and/or that has a lower molecular weight than that produced by wild-type bacteria of the same serotype or strain.

### CpsC

Full length capsular polysaccharide biosynthesis protein CpsC (NCBI Reference Sequence: YP_007968737.1) from Streptococcus has the following amino acid sequence (SEQ ID NO:1)

MNKIANTEVEINIFNLLKKLWKKKFLIIFVAIAFATAGLFYRLFIVTPQYTSSTRIYVS
KPTLPNNSITVQDLQAGSFLANDYKEIITSTDVLEKVISSEKLNYPSSQLLQKITVS
ILKGTRVISISVEDVNPKMSQKIANSVREAAVSKIKAVTQVEDITTIEKGNLPKSPS
SPNIKKNVLIWFIVGAGLSTIVLVIMGILDDRVNTEEDIEKVLGLTSLGIVPDLNKL

The protein, which may also be referred to as "Tyrosine-protein kinase transmembrane modulator EpsC", has the following polynucleotide sequence (SEQ ID NO:2):

ATGAATAAAATAGCTAATACAGAAGTTGAAATAAATATATTCAACCTTTTAAAA
AAATTGTGGAAAAAGAAATTTTTAATCATATTTGTTGCCATTGCGTTTGCAAC
AGCTGGACTTTTCTATAGACTTTTTATTGTTACGCCACAATATACTTCTTCAAC
AAGGATATATGTCAGTAAACCTACTCTCCCTAATAATAGTATTACTGTACAAG
ATCTACAAGCAGGGAGTTTTCTTGCAAATGACTATAAGGAGATTATTACGTCT
ACTGACGTTCTAGAAAAAGTTATTTCTTCTGAAAAATTGAATTATCCTTCGTCT
CAGTTGCTGCAAAAAATAACAGTTTCTATTTTAAAAGGTACACGTGTTATTTC
AATATCGGTCGAAGATGTTAATCCAAAAATGTCTCAAAAAATAGCAAATTCAG
TTAGAGAAGCAGCAGTTTCAAAAATCAAGGCAGTTACTCAAGTAGAGGATAT
CACTACTATTGAGAAGGGAAATTTACCTAAATCACCATCTTCTCCTAATATTA
AAAAGAATGTACTAATCTGGTTTATTGTTGGTGCAGGATTATCAACTATTGTT
TTAGTTATTATGGGTATTTTGGATGATCGTGTTAATACTGAAGAAGATATTGA
AAAGGTCCTAGGACTTACTTCTTTAGGTATAGTACCAGATTTGAATAAACTTT
AA

The inventors have discovered that bacterial mutants which comprise a deletion of the extracellular domain of CpsC produce capsular polysaccharide which has a smaller chain length and is of a lower molecular weight than capsular polysaccharides produced by native, wild-type bacteria of the same serotype.

In certain embodiments, the mutation comprises or consists of a deletion of the part of polynucleotide sequence that encodes the extracellular domain of CpsC. In certain embodiments, the mutation results in deletion of the entire CpsC polypeptide. Particular mutations comprise a deletion of all or part of the domain from an amino acid selected from the group consisting of amino acids 25, 26, 27, 28, 29, 30, 31, 32, 33, 34, 35, 36, 37, 38, 39, 40, 41, 42, 43, 44, 45, 46, 47, 48, 49 and 50 to an amino acid selected from the group consisting of amino acids 178, 179, 180, 181, 182, 183, 184, 185, 186, 187, 188, 189, 190, 191, 192, 193, 194, 195, 196, 197, 198, 199, 200, 202 and 202 numbered according to SEQ ID NO: 1. Particular mutations include a deletion of from amino acid 45 to amino acid 181, amino acid 46 to amino acid 180 or amino acid 25 to amino acid 202 numbered according to SEQ ID NO: 1, for example a deletion of SEQ ID NO:3. Other possible mutations include a deletion of one or more parts of the extracellular domain selected from the group consisting of the Wzz motif (SEQ ID NO:8), Nuf2 family motif (SEQ ID NO:9) and DUF59 motif (SEQ ID NO:10). Similar deletions and mutations of CpsC that impact the capsular polysaccharide chain length may be identified, for example having sequence identity with CpsC mutations of the invention of more than 90%, 91%, 92%, 93%, 94%, 95%, 96%, 97%, 98%, 99% or greater. The recombinant *Streptococcus agalactiae* strains may produce capsular polysaccharide having a molecular weight and/or length that is less than 10%, 15%, 20%, 25%, 30%, 35%, 40%, 45%, 50%, 55%, 60%, 70%, 75%, 80%, 85%, 95% than that of a wild-type cell of the same serotype.

### Variants

Variants of CpsC from other strains of *Streptococcus agalactiae* are known and can be easily identified by, for example, use of BLAST searches of the above sequences. Variants of CpsC include by way of non-limiting example, Uniprot accession numbers: R4Z8W1, K4PVK0, S9KAC9, W2BNQ3, Q3DHZ2 and the like. Thus, the invention is also applicable to allelic variants of the disclosed CpsC proteins from *Streptococcus agalactiae.* In some embodiments, the degree of sequence identity is greater than 80%, 90%, 91%, 92%, 93%, 94%, 95%, 96%, 97%, 97.5%, 98%, 98.5%, 99%, 99.5% or more, particularly 93% or more, yet more particularly 94% or more. These polypeptides include homologs, orthologs, allelic variants and functional mutants. Typically, 50% identity or more between two polypeptides is considered to be an indication of functional equivalence.

In the context of the present invention, the term "capsular polysaccharide" is intended to mean the capsular polysaccharides of *Streptococcus agalactiae* In GBS, one of the most important virulence factors is the capsular polysaccharide. To date, ten capsular capsule polysaccharide serotypes have been found: la, Ib, II, III, IV, V, VI, VII, VIII and IX.

### Capsular saccharides

The capsular saccharide of *Streptococcus agalactiae* is covalently linked to the peptidoglycan backbone of GBS, and is distinct from the group B antigen, which is another saccharide that is attached to the peptidoglycan backbone. The GBS capsular saccharides are chemically related, but are very different from an antigenic standpoint. All GBS capsular polysaccharides share the following trisaccharide core:
B-D-GlcpNAc(1→3)β-D-Galp(1→4)β-D-Glcp

The various GBS serotypes differ by the way in which this core is modified. The difference between serotypes la and III, for instance, arises from the use of either the GlcNAc (Ia) or the Gal (III) in this core for linking consecutive trisaccharide cores. Serotypes la and Ib both have a [α D NeupNAc(2→3)β D Galp (1→] disaccharide linked to the GlcNAc in the core, but the linkage is either 1 →4 (Ia) or 1→3 (Ib). GBS-related diseases arise primarily from serotypes la, Ib, II, III, IV, V, VI, VII, and VIII, with over 85% being caused by five serotypes: la, Ib, II, III & V. The invention preferably relates to a saccharide from one or more of these five serotypes, particularly from one or more of serotypes II and V. The capsular saccharides generally include: (a) a terminal N-acetyl-neuraminic acid (NeuNAc) residue (commonly referred to as sialic acid), which in all cases is linked 2→3 to a galactose residue; and (b) a N-acetyl-glucosamine residue (GlcNAc) within the trisaccharide core.

When the GBS saccharide has been purified by base extraction, then O-acetylation is typically lost. Particularly, capsular polysaccharides extracted by the methods of the invention are fully O-acetylated and/or N-acetylated are not de- acetylated (partially or fully). The effect of de-acetylation etc. can be assessed by routine assays.

Particularly the degree of sialic acid oxidation of the GBS capsular polysaccharide is not less than 10%, 20%, 30%, 40%, 50%, 60%, 70%, 80%, 85%, 90%, 95% and particularly the N-acetyl-neuraminic acid (NeuNAc or sialic acid) content of the GBS serotype V capsular polysaccharide is greater than 50%, greater than 60%, greater than 70%, greater than 75%, greater than 80%, greater than 85%, greater than 90%, greater than 95%, when compared to native GBS serotype polysaccharide wherein the NeuNAc content is considered to be about 100%. Particularly, the GBS polysaccharide is a fully sialylated or "native polysaccharide". For example, with a sialic acid content of about 100%, 99%, 98%, 97%, 96%, 95%, 94%, 93%, 92%, 91 %, about 90% (or any range between these values) when compared to native GBS polysaccharide.

The saccharide purified according to the invention is shorter than the GBS polysaccharide found in nature or isolated from a wild-type bacterium.

### Purification

Capsular polysaccharide may be extracted and purified from CpsC mutants by techniques known in the art, for example, as disclosed in EP2270056 or EP1051506 herein incorporated by reference. For example:
Starting material: Generally the starting materials for extracting CPS will be bacterial cells, for example, homogenized bacterial cells wherein the cells are comprise a mutation in the CpsC gene. Cells may be separated by centrifugation or microfiltration. In some embodiments the concentrated supernatant, typically 1 0-15 fold, may be used. Preferably the CPS will be present at a concentration of about 5-20 mg/ml.

Base extraction: Isolated bacterial cells can be contacted with a variety of bases to extract the CPS. Non-limiting examples of bases which may be used according to this invention are NaOH, KOH, LiOH, NaHCO₃, Na₂CO₃, K₂CO₃, KCN, Et₃N, NH₃, H₂N₂H₂, NaH, NaOMe, NaOEt or KOtBu.

Alcoholic precipitation and cation exchange: The GBS capsular saccharide obtained following cell inactivation/lysis will generally be impure and may be contaminated with bacterial nucleic acids and proteins. The process of the invention utilises alcoholic precipitation. Since base extraction is not used, materials will not need to be neutralised prior to the precipitation, again decreasing the length of time taken to purify the capsular polysaccharide.

The alcohol used to precipitate contaminating nucleic acids and/or proteins is preferably a lower alcohol, such as methanol, ethanol, propan-1-ol, propan-2-ol, butan-1-ol, butan-2-ol, 2-methyl-propan-1-ol, 2-methyl-propan-2-ol, diols, etc. The selection of an appropriate alcohol can be tested empirically, without undue burden, but alcohols such as ethanol and isopropanol (propan-2-ol) are preferred, rather than alcohols such as phenol.

The alcohol is preferably added to the polysaccharide suspension to give a final alcohol concentration of between 10 percent and 50 percent (e.g. around 30 percent). The most useful concentrations are those which achieve adequate precipitation of contaminants without also precipitating the polysaccharide. The optimum final alcohol concentration may depend on the GBS serotype from which the polysaccharide is obtained, and can be determined by routine experiments without undue burden. Precipitation of polysaccharides as ethanol concentrations>50 percent has been observed.

The alcohol may be added in pure form or may be added in a form diluted with a miscible solvent (e.g. water). Preferred solvent mixtures are ethanol:water mixtures, with a preferred ratio of between around 70:30 and around 95:5 (e.g. 75:25, 80:20, 85:15, 90:10).

The saccharide is also treated with an aqueous metal cation. Monovalent and divalent metal cations are preferred, and divalent cations are particularly preferred, such as Mg++, Mn++, Ca++, etc, as they are more efficient at complex formation. Calcium ions are particularly useful, and so the alcohol mixture preferably includes soluble calcium ions. These may be added to a saccharide/ alcohol mixture in the form of calcium salts, either added as a solid or in an aqueous form. The calcium ions are preferably provided by the use of calcium chloride.

The calcium ions are preferably present at a final concentration of between 10 and 500 mM e.g. about 0.1 M. The optimum final Ca++ concentration may depend on the GBS serotype from which the polysaccharide is obtained, and can be determined by routine experiments without undue burden.

The alcohol and the cation play different roles (the alcohol is used to precipitate contaminants, whereas the cation stabilises and complexes the saccharide in soluble form) but produce a combined effect. Although the aim is to prepare a mixture of the saccharide, the alcohol and the cation, these three components need not be mixed together simultaneously. Thus the alcohol and cation can be used sequentially or simultaneously. Sequential treatment is preferred, and a particularly preferred process involves addition of the cation to the saccharide followed by addition of the alcohol to the cation/saccharide mixture, although the alcohol can be used before the cation if desired.

After alcoholic precipitation of contaminating proteins and/or nucleic acids, the GBS capsular polysaccharide is left in solution. The precipitated material can be separated from the polysaccharide by any suitable means, such as by centrifugation. The supernatant can be subjected to microfiltration, and in particular to dead-end filtration (perpendicular filtration) in order to remove particles that may clog filters in later steps (e.g. precipitated particles with a diameter greater than 0.22 micrometres). As an alternative to dead-end filtration, tangential microfiltration can be used.

Diafiltration: The process of the invention may involve a step of filtration, for example, diafiltration after the precipitation of proteins and/or nucleic acids. Tangential flow diafiltration may be used. The filtration membrane should thus be one that allows passage of impurities while retaining the capsular polysaccharide. A cut-off in the range 10kDa-30kDa is typical. Smaller cut-off sizes can be used but higher cut-off sizes advantageously allow removal of other contaminants without leading to loss of the capsular saccharide. At least 1 cycle of diafiltration may be performed e.g. 2, 3, 4, 5, 6, 7, 8, 9, 10, 11 or more.

In some embodiments, the method further comprises a step of removing contaminating nucleic acids and/or proteins. Particularly contaminating nucleic acids and/or proteins may be removed by the use of precipitation. Yet more particularly, contaminating nucleic acids and/or proteins may be removed from the capsular polysaccharide in aqueous form by the use of alcoholic precipitation. When a step of alcoholic precipitation is included, an alcohol and an aqueous metal cation may be used to precipitate the nucleic acids and/or proteins leaving the polysaccharide in solution. When a step of alcoholic precipitation is included, the method may include a further step of separating the precipitated material from the polysaccharide. Particularly the precipitated material may be separated from the polysaccharide by filtration and yet more particularly the method may comprise a step of diafiltration after the precipitation of nucleic acids and/or proteins. In some embodiments the alcohol is ethanol or isopropanol. In some embodiments the aqueous metal cation is CaCl₂. Particularly, methods of the invention will comprise one or more steps of filtration, for example, ultrafiltration and/or gel filtration. In particular embodiments, gel filtration using Sephacryl ® is performed, for example using Sephacryl ® S-500 gel.

Further treatment of the capsular polysaccharide: The polysaccharide may be further treated to remove contaminants. This is particularly important in situations where even minor contamination is not acceptable (e.g. for human vaccine production). For example, further precipitation steps may be used. Where an aqueous re-solubilisation was performed then this precipitation will typically use an alcohol, as described in the preceding section; conversely, where an alcoholic re-solubilisation was performed then this precipitation will typically use an aqueous cation solution, as described in the preceding section. The precipitated saccharide can then be separated from any remaining aqueous contaminants e.g. by centrifugation. The precipitated material is stable and can be stored for future use. Further rounds of precipitation and filtration can also be performed. Depth filtration can also be used e.g. as an alternative to centrifugation. Depth filtration will typically be used after solubilisation in alcohol.

The precipitated material may be subjected to vacuum drying. This treatment will typically be used not to stabilise the saccharide for storage, but to dry the saccharide and remove any residual alcohol.

The purified capsular polysaccharide of the invention can be used as an antigen without further modification e.g. for use in in vitro diagnostic assays, for use in immunisation, etc. For immunisation purposes, however, it is preferred to conjugate the saccharide to a carrier molecule, such as a protein. In general, covalent conjugation of saccharides to carriers enhances the immunogenicity of saccharides as it converts them from T-independent antigens to T-dependent antigens, thus allowing priming for immunological memory. Conjugation is a well known technique. Thus the methods of the invention may include the further step of conjugating the purified saccharide to a carrier molecule. The invention may also provide processes for preparing pharmaceutical compositions, comprising the steps of mixing (a) a polysaccharide of the invention (optionally in the form of a conjugate) with (b) a pharmaceutically acceptable carrier. Typical 'pharmaceutically acceptable carriers' include any carrier that does not itself induce the production of antibodies harmful to the individual receiving the composition. Suitable carriers are typically large, slowly metabolised macromolecules such as proteins, polysaccharides, polylactic acids, polyglycolic acids, polymeric amino acids, amino acid copolymers, lactose, and lipid aggregates (such as oil droplets or liposomes). Such carriers are well known to those of ordinary skill in the art. The vaccines may also contain diluents, such as water, saline, glycerol, etc. Additionally, auxiliary substances, such as wetting or emulsifying agents, pH buffering substances, and the like, may be present. Sterile pyrogen-free, phosphate-buffered physiologic saline is a typical carrier. The pharmaceutical compositions may be packaged into vials or into syringes. The syringes may be supplied with or without needles. A syringe will include a single dose of the composition, whereas a vial may include a single dose or multiple doses. Aqueous compositions of saccharides of the invention are suitable for reconstituting other vaccines from a lyophilised form. Where a composition of the invention is to be used for such extemporaneous reconstitution, the invention provides a process for reconstituting such a lyophilised vaccine, comprising the step of mixing the lyophilised material with an aqueous composition of the invention. The reconstituted material can be used for injection.

### General

The term "comprising" encompasses "including" as well as "consisting" e.g. a composition "comprising" X may consist exclusively of X or may include something additional e.g. X + Y. The term "consisting essentially of" means that the composition, method or structure may include additional ingredients, steps and/or parts, but only if the additional ingredients, steps and/or parts do no materially alter the basic and novel characteristics of the claimed composition, method or structure. The term "consisting of" is generally taken to mean that the invention as claimed is limited to those elements specifically recited in the claim (and may include their equivalents, insofar as the doctrine of equivalents is applicable).

The term "about" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

The word "substantially" does not exclude "completely" e.g. a composition which is "substantially free" from Y may be completely free from Y. For example, "substantially free" from Y can be understood as a composition containing not more than 5% Y, not more than 4% Y, not more than 3% Y, not more than 2% Y, not more than 1% Y, or not more than 0.1 % Y. Where necessary, the word "substantially" may be omitted from the definition of the invention.

As used herein, unless otherwise clear from context the term "or" is understood to be inclusive and can be used interchangeably with the term "and/or".

The term "mutant" refers to a gene or gene product that displays modifications in sequence and/or functional properties (i.e., altered characteristics) when compared to the wild-type gene or gene product.

All GenBank Accession numbers provided herein are incorporated by reference in the version available on the date of filing the instant application.

The term "recovering" means the isolation of the capsular polysaccharide in different purities, for example between 5% and 100% purity, preferred purities are in the range of 10% and 99%, 20% and 99%, 30% and 99%, 40% and 99%, 50% and 99%, 60% and 99%, 70% and 99%, 80% and 99%, 90% and 99%. Particular purities are greater than 20%, 30%, 40%, 50%, 60%, 70%, 80%, 90%, 95% or 99%. Recovering may also be referred to as extracting or purifying. The term "purity" takes the general meaning used in the art to refer to the percentage of the in-hand, isolated sample is actually capsular polysaccharide.

This invention is further illustrated by the following examples which should not be construed as limiting.

### EXAMPLES

### Bacterial strains and growth conditions.

GBS 515 (Wessels, et al. 1993) and its isogenic derivatives were grown in Todd-Hewitt broth (THB medium; Difco Laboratories) at 37°C, 5% CO₂. Tryptic soy broth (Difco Laboratories), 15 g/L agar (TSA) was used as solid medium. Strains were stored at -80°C in THB medium + 15% glycerol. MAX Efficiency® DH5α™ Competent Cells (Invitrogen) and competent HK100 *E.coli* cells prepared in house were used for transformation, propagation, and preparation of plasmids. *E*. *coli* was grown at 37°C with agitation (180 rpm) in Luria-Bertani (LB, Difco laboratories) broth, or on 15 g/L agar plates (LBA). Erythromycin (Erm) was used for selection of GBS (1 µg/ml) or *E*. *coli* (100 µg/ml) containing the pJRS233-(Perez-Casal et al. 1993) derived plasmids used for mutagenesis. Kanamycin (Kan) was used for selection *E*. *coli* (50 µg/ml) containing the pET24b-derived plasmids (Novagen) used for initial mutagenesis of inserts, prior to transfer into pJRS233.

### Construction of CpsC plasmids for mutagenesis and chromosomal complementation

To prepare each mutant strain, the shuttle vector pJRS233 (Perez-Casal J, *et al.* 1993) containing the gene locus with an in-frame deletion or a codon substitution was constructed. Mutant strains developed are described in the Table 1:

| **Mutant name** | **Description** |
|---|---|
| **Δ*cpsC*** | *cpsC* deletion |
| **CpsC(Δext_domain)** | deletion of the CpsC extracellular domain |
| **CpsC(ΔC-ter,)** | Deletion of the CpsC intracellular domain |

Constructs for genes with codon substitutions were prepared using a splicing by overlap extension PCR (SOEing-PCR) strategy. Briefly, the two parts of the gene up- and downstream of the codon substitution were amplified from 515 gDNA using the PfuUltra II Fusion HS DNA Polymerase (Agilent Technologies). 900-1000 bp upstream and downstream the coding sequence of the gene were added to the inserts. Primers used to amplify the two parts of the genes have 15 bp overlapping tails and introduce the codon substitution in the two PCR products that are then joined together by SOEing-PCR. The resulting fragment was ligated into pJRS233 using BamHI and Xhol restriction sites.

Constructs for genes with in-frame deletions were prepared using the Polymerase Incomplete Primer Extension (PIPE) method. Briefly, the gene plus 900-1000 bp up- and downstream the coding sequence were amplified from 515 gDNA and cloned into pET24b using Notl and Xhol (*cpsA* inserts) or BamHI and Xhol (*cpsD* inserts) restriction sites. In frame deletions of the genes were developed by amplifying the plasmid using primers with 15 bp overlapping tails annealing at the two sides of the region to delete. Linear plasmids were transformed into HK100 competent cells able to re-circularize the plasmid. The resulting inserts were then transferred into pJRS233 plasmid by restriction digestion and ligation.

Constructs for chromosomal complementation were prepared transferring into pJRS233 the wild type inserts cloned into pET24b.

### Construction of isogenic mutants and chromosomally complemented strains

An insertion/duplication and excision mutagenesis strategy was used both to obtain the in-frame deletion/codon substitution in the genes and to replace the mutations to obtain the chromosomally complemented strains. Briefly, pJRS233-derived plasmids purified from *E. coli* were used to transform electrocompetent 515 cells by electroporation. Transformants were selected by growth on TSA + Erm at 30°C for 48 hours. Integration was performed by growth of transformants at 37°C (non-permissive temperature for the suicide shuttle vector) with Erm selection. Excision of the integrated plasmid was performed by serial passages in THB at 30°C, and parallel screening for Erm-sensitive colonies on plate. Mutants were verified by PCR sequencing of the loci.

To obtain the chromosomally complemented strains, pJRS233-derived plasmids containing the wild type version of the genes and the flanking 900-1000 bp up-and downstream were purified from *E. coli* and complementation of the respective mutant strains was performed as described for mutagenesis.

### qRT-PCR analysis

Bacteria were harvested at two time points: at OD₆₀₀=0.4 (log phase) and OD₆₀₀=1.7 (early stationary phase). To rapidly arrest transcription, 10 ml of bacteria were cooled on ice and added to 10 ml of frozen THB medium in a 50 ml conical tube. GBS cells were then collected by centrifugation for 15 min at 4000 rpm, 4°C, and resuspended in 800 µl of TRlzol (Invitrogen). Bacteria were disrupted mechanically by agitation with Lysing matrix B in 2 ml tubes (DBA Italy) using a homogenizer (Fastprep-24, Millipore) for 60 sec at 6.5 m/s for two cycles, and kept on ice for 2 min between the cycles. Samples were then centrifuged for 5 min at 8000 x *g*, 4°C and RNA was extracted with Direct-zol™ RNA MiniPrep kit (Zymo Research) according to the manufacturer's instructions. RNA samples were treated with DNase (Roche) for 2 h at 37°C and further purified using the RNA MiniPrep kit (Qiagen), including a second DNase treatment on the column for 30 min at room temperature (RT), according to the manufacturer's instructions. cDNA was prepared using the Reverse Transcription System (Promega) by using 500 ng of RNA per reaction. Real time quantitative PCR (qRT-PCR) was performed on 50 ng of cDNA that was amplified using LightCycler^{®} 480 DNA SYBR Green I Master, (Roche). Reactions were monitored using a LightCycler^{®} 480 instrument and software (Roche). Three technical replicates were monitored for each strain/condition analyzed. To quantify *cps* operon transcription level primers annealing on *cpsA* and *cpsE* were used respectively for *cpsA* mutants. The transcript amounts in each condition were standardized to an internal control gene (*gyrA*) and compared with standardized expression in the wild-type strain (ΔΔC_{T} method).

### Quantification of the capsular polysaccharide attached to the cell surface

An overnight culture was used to inoculate (1:1000) 50 ml of fresh THB and bacteria were grown at 37°C for 8 hours. GBS cells were collected by centrifugation for 15 min at 4000 rpm at 4°C, resuspended in 1.1 ml of PBS + 0.8 M NaOH and incubated at 37°C for 36 hours. Samples were neutralized and pelleted by centrifugation for 10 min at 4000 rpm, 4°C. 850 µl of the supernatant were diluted in 7.15 ml of water, and centrifuged for 10 min at 4000 rpm at 4°C. 7.2 µl of the supernatant were loaded on a Vivaspin 10 tube (Sartorius Stedim Biotech) which was centrifuged at 4000 rpm until most of the solution passed through the membrane. After two washes with 1 ml of water, the CPS extract was recovered from the membrane and resuspended in 1.6 ml of water. The amount of CPS present in the extract was estimated by measuring the sialic acid content using the colorimetric resorcinol-hydrochloric acid method (Svennerholm *et al,* 1957). Briefly, 120 µl of extract were mixed with 380 µl of water and 500 µl of freshly prepared solution R3 (resorcinol 0.2%, copper sulfate 0.3 mM, HCl 30% in H₂O). Samples were boiled for 20 min and then cooled to room temperature before being transferred into 1 ml cuvettes to measure their absorbance at 564 nm. The sialic acid content of the samples was then calculated using a concomitantly prepared standard curve using serial dilutions of purified sialic acid. CPS extracts were prepared three times from independent growths to minimize the biological variability.

### Quantification of the capsular polysaccharide released in the growth medium

An overnight culture was used to inoculate (1:1000) 10 ml of fresh THB and bacteria were grown at 37°C for 8 hours. GBS cells were pelleted by centrifugation for 15 min at 4000 rpm at 4°C, and the growth medium was collected and filtered using a 0.22 µm Nalgene Syringe Filter (Thermo Scientific). The amount of capsular polysaccharide released in the growth medium was estimated by dot blot. Purified serotype la CPS 10 mg/ml was used as standard. Eight serial dilutions were prepared in a 96-well plate by diluting the standard and the growth media in PBS (dilution ratios 1:2 for media, 1:4 for the standard). 2 µl of each serial dilution were spotted onto a nitrocellulose membrane. The membrane was dried for 20 min and blocked by soaking in 5% (w/v) skim milk in PBS-Tween 0.05%. The membrane was then probed with a primary mouse monoclonal anti- serotype la CPS-CRM conjugated antibody (30E9/B11) used at 1:2000, washed 3 times in PBS-Tween 0.05%, and incubated in 1:15000 of secondary goat anti-mouse antibody conjugated to horseradish peroxidase. Detection was performed using Thermo Scientific Pierce ECL Western Blotting Substrate according to the manufacturer's instructions.

### Western blot on the capsular polysaccharide released in the growth medium

20 µl of medium were mixed with 10 µl of 3x NuPAGE® LDS Sample Buffer + Reducing Agent and boiled for 5 min. Then, 20 µl were loaded on a NuPage 4-12% Bis-Tris Gel 1.0 mm, 12 well (Life Technologies) (running buffer: MOPS 1x) and run at 150V until the band corresponding to 28 kDa of the SeeBlue® Plus2 Pre-stained Protein Standard (Life Technologies) reached the bottom of the gel. The samples separated on the gel were transferred to a nitrocellulose membrane using the iBlot® 7-Minute Blotting System (Life Technologies). The membrane was blocked blocked by soaking in 5% (w/v) skim milk in PBS-Tween 0.05%. The membrane was then probed with a primary mouse monoclonal anti- serotype la CPS-CRM conjugated antibody (30E9/B11) used at 1:2000, washed 3 times in PBS-Tween 0.05%, and incubated in 1:15000 of secondary goat anti-mouse antibody conjugated to horseradish peroxidase. Detection was performed using Thermo Scientific Pierce ECL Western Blotting Substrate according to the manufacturer's instructions.

Those skilled in the art will recognize, or be able to ascertain using no more than routine experimentation, many equivalents to the specific embodiments of the invention described herein. Such equivalents are intended to be encompassed by the following claims.

### REFERENCES

Wessels, M. R., Paoletti, L. C., Rodewald, A. K., Michon, F., DiFabio, J., Jennings, H. J. & Kasper, D. L. (1993) Infect. Immun. 61, 4760-4766.
Perez-Casal J, Price JA, Maguin E, Scott JR. (1993) Mol Microbiol 8, 809-819.
Svennerholm L., (1957) Biochim Biophys Acta. 24(3), 604-611.

## Claims

1. A method for producing a low molecular weight capsular polysaccharide comprising: culturing a CpsC mutant bacterium in a suitable culture medium and isolating the capsular polysaccharide from the bacterium.

2. The method of claim 1 wherein the CpsC mutant bacterium is derived from *Streptococcus agalactiae.*

3. The method of claim 1 or 2 wherein the CpsC mutant bacterium comprises an altered CpsC protein wherein the nucleotide sequence that encodes the extracellular domain of CpsC protein (SEQ ID NO:1) is deleted or partially deleted and wherein the CpsC mutant bacterium produces low molecular weight capsular polysaccharide.

4. The method of claim 3, wherein the capsular polysaccharide is from a *Streptococcus agalactiae* serotype selected from the group consisting of la, Ib, II, III, IV, V, VI, VII or VII particularly from serotypes la, Ib, II, III or V.

5. The method of any preceding claim, wherein the low molecular weight polysaccharide has a molecular weight of between 1 OkDa and 70kDa.

6. The method of claim 5 wherein the step of isolating the capsular polysaccharide from the bacterium comprises:
(i) providing for extraction isolated bacterial cells, concentrated bacterial supernatants from homogenized bacterial cells or pelleted cells;
(ii) extracting the capsular polysaccharide from cellular components, particularly by contacting the isolated bacterial cells, concentrated bacterial supernatants from homogenised bacterial cells or pelleted cells with a base reagent; and
(iii) separating the extracted capsular polysaccharide from the cellular components to recover the purified capsular polysaccharides.

7. The process of claim 6, wherein step (iii) comprises removing contaminating nucleic acids and/or proteins from the capsular polysaccharide in aqueous form by the use of alcoholic precipitation, wherein an alcohol and an aqueous metal cation are used to precipitate the nucleic acids and/or proteins leaving the polysaccharide in solution and, separating the precipitated material from the polysaccharide.

8. The method of claim 7, wherein the process further comprises a step of diafiltration after the precipitation of nucleic acids and/or proteins.

9. The method of claim 7 or 8, wherein the alcohol is ethanol or isopropanol.

10. The method of any one of claims 7 to 9, wherein the aqueous metal cation is CaCl₂.

11. The process of any one of claims 7 to 10, further comprising one or more steps of filtration.

12. A low molecular weight capsular polysaccharide from *Streptococcus agalactiae*, obtainable by the method of any preceding claim.

13. An immunogenic composition, particularly a vaccine composition, comprising the low molecular weight capsular polysaccharide of claim 12.
